# EUROPÄISCHE PATENTSCHRIFT

(11) **EP 0 772 602 B1**
(45) Veröffentlichungstag und Bekanntmachung des Hinweises auf die Patenterteilung: **20.09.2000**
(21) Anmeldenummer: 95926920.0
(22) Anmeldetag: 18.07.1995
(51) Int. Cl.: C07D 233/58

(54) **VERFAHREN ZUR HERSTELLUNG 1,3-DISUBSTITUIERTER IMIDAZOLIDINONE**
PROCESS FOR PREPARING 1,3-DISUBSTITUTED IMIDAZOLIDINONES
PROCEDE DE PREPARATION D'IMIDAZOLIDINONES DISUBSTITUEES EN 1,3

(30) Priorität: 20.07.1994 DE 4425696
(43) Veröffentlichungstag der Anmeldung: 14.05.1997
(73) Patentinhaber: BASF AKTIENGESELLSCHAFT, 67056 Ludwigshafen (DE)
(72) Erfinder: HENKELMANN, Jochem, D-68165 Mannheim (DE); RÜHL, Thomas, D-67227 Frankenthal (DE)
(74) Vertreter: Isenbruck, Günter, Dr.
(86) Internationale Anmeldenummer: EP9502806
(87) Internationale Veröffentlichungsnummer: WO9602516

(56) Entgegenhaltungen:
- US-A- 2 892 843
- J. ORG. CHEM. USSR (ENGL. TRANSL.), 1983 Seiten 436-439, V. V. MIKHEEV ET AL. 'Reaction of organic carbonates with amines. III. Reaction of amines with alkylene carbonates'
- J. AMER. CHEM. SOC., Bd. 79, 1957 Seiten 672-675, E. DYER, H. SCOTT 'The Preparation of Polymeric and Cyclic Urethanes und Ureas from Ethylene Carbonate and Amines'

## Beschreibung

Die vorliegende Erfindung betrifft die Herstellung 1,3-disubstituierter Imidazolidin-2-öne aus 1,3-Dioxalan-2-on (Ethylencarbonat) und primären Aminen.

1,3-disubstituierte Imidazolidin-2-one, insbesondere 1,3-Dimethylimidazolidin-2-on, sind interessante polare, nicht-protische Lösungsmittel insbesondere für hochmolekulare Verbindungen wie Polyamide, Polyvinylchlorid, Polyvinylalkohol, Polystyrol, Polyurethan und Phenolharze. Weiter werden 1,3-disubstituierte Imidazolidin-2-one in der Textilindustrie als Appreturmittel verwendet.

Die Synthese 1,3-disubstituierter Imidazolidinone ist bereits vielfach beschrieben worden. Die katalytische Hydrierung von 1,3-Dimethoxymethyl- bzw. intermediär gebildetem 1,3-Dihydroxymethylimidazolidin-2-on und anderen N,N'-Dihydroxymethyl-substituierten cyclischen Harnstoffen (US-A 2,422,400; US-A 4,617,400) verläuft unter starker Rückstandsbildung. Die Alkylierung cyclischer Harnstoffe (Synthesis (1982), 464; J. Med. Chem. 24, 1089 (1981)) benötigt neben der stöchiometrischen Menge einer Base toxikologisch bedenkliche Alkylhalogenide. Die Umsetzungen von N,N'-disubstituierten Diaminen mit Harnstoff (EP-A 198 345), die in Lösungsmitteln durchgeführt werden muß, oder mit dem toxikologisch bedenklichen Phosgen (EP-A 183 076) sind ebenfalls bekannt. Weiter beschrieben sind die Synthesen aus Kohlendioxid, primärem Amin und Ethylenglykol (JP-A 5 9155-364) bzw. Dichlorethan sowie die Leukart-Wallach-Reaktion zur Synthese von 1,3-Dimethylimidazolidinon, die einen heterogenen Hydrierkatalysator erfordert (GB-B1 517 820; DE-C 1 545 614; US-A 2,422,400). Aus der US-A-2 892 843 ist die Herstellung von unsubstituierten Alkylenharnstoffen aus Dioxolanen und Ammoniak bekannt.

Aufgabe der Erfindung war es, einen Syntheseweg für 1,3-disubstituierte Imidazolidin-2-one zu entwickeln, der einfach und wirtschaftlich ist. Insbesondere soll das Verfahren unter Umweltgesichtspunkten bekannten Synthesewegen überlegen sein.

Diese Aufgabe wird durch ein Verfahren gelöst, wie es in den Ansprüchen definiert ist. Bevorzugte Ausführungsformen sind in den Unteransprüchen und in der folgenden Beschreibung angegeben.

Überraschenderweise wurde gefunden, daß sich Ethylencarbonat mit primären Aminen direkt zum gewünschten cyclischen Harnstoff umsetzen läßt, ohne daß Lösungsmittel oder Katalysatoren erforderlich sind.

Erfindungsgemäß wird also ein Verfahren zur Herstellung von Imidazolidin-2-onen der allgemeinen Formel (I) 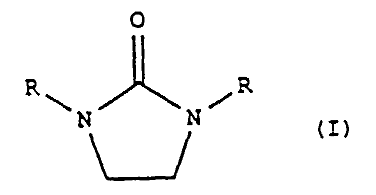 in der die Reste R, die gleich oder verschieden sein können,
a) geradkettige, verzweigte oder cyclische Alklylreste mit 1 bis 12 C-Atomen, die ihrerseits mit C₆₋₁₀-Aryl, F, Cl, Br substituiert sein können;
b) Arylreste mit 6 bis 10 C-Atomen, die ihrerseits mit C₁₋₁₂-Alkyl substituiert sein können;
c) Heteroalkylreste, in denen die wie oben definierten Alkylreste durch ein oder mehrere Heteroatome, ausgewählt aus O, S und N, unterbrochen sind,
d) Heteroarylreste mit 5 bis 10 Ringatomen, die 1 bis 3 Heteroatome, ausgewählt aus O, S, N, enthalten,
darstellen, dadurch gekennzeichnet, daß man 1,3-Dioxalan-2-on (Ethylencarbonat) mit mindestens einer Verbindung der allgemeinen Formel (II)

RNH₂ (II)

umsetzt, in der R die vorstehende Bedeutung aufweist. Ein bevorzugtes Verfahrensprodukt ist 1,3-Dimethylimidazolidin-2-on, d.h. R ist dann in beiden Fällen Methyl.

Die Reaktion wird im allgemeinen bei 150°C bis 300°C, bevorzugt bei 200°C bis 250°C, besonders bevorzugt unter Eigendruck, durchgeführt. Der sich einstellende Eigendruck liegt gewöhnlich je nach Amin zwischen 50 und 150 bar.

Die Menge an eingesetztem primärem Amin in bezug auf Ethylencarbonat ist vorteilhaft stöchiometrisch oder überstöchiometrisch, z.B. 5fach stöchiometrisch, bevorzugt im Bereich eines 1,5- bis 3fachen stöchiometrischen Überschusses.

Eine Reaktionszeit von bis zu etwa 24 Stunden hat sich häufig als ausreichend erwiesen.

Unter Verwendung von Wasser läßt sich die Ausbeute des Verfahrens im allgemeinen steigern. Die eingesetzte Wassermenge kann bis zu 50 Gew.-%, vorteilhaft 30 bis 45 Gew.-%, bezogen auf eingesetztes Ethylencarbonat, betragen.

Die Aufarbeitung des Reaktionsgemischs kann durch einfache Destillation des Rohproduktes erfolgen.

### Beispiel 1

In einen Autoklaven wurden bei Raumtemperatur 500 g Ethylencarbonat, 500 g Methylamin und 200 g Wasser eingetragen und 24 Stunden bei 250°C erhitzt. Der Eigendruck der Reaktion erreichte 120 bar.

Anschließend wurde der Ansatz abgekühlt, entspannt und destilliert. Bei 2 mbar Druck und 60°C wurden 550 g 1,3-Dimethylimidazolidin-2-on (R = CH₃ in Formel (I)) (85% d. Th.) erhalten.

### Beispiel 2

Der gleiche Ansatz wie in Beispiel 1 wurde ohne Wasser 24 Stunden bei 250°C erhitzt. Die anschließende Aufarbeitung ergab 476 g 1,3-Dimethylimidazolidin-2-on (75% Ausbeute).

### Beispiel 3

500 g Ethylencarbonat wurden wie in Beispiel 1 mit 350 g Methylamin umgesetzt und aufgearbeitet. Die Ausbeute an 1,3-Dimethylimidazolidin-2-on betrug 55%.

### Beispiel 4

Ethylencarbonat und Methylamin wurden wie in Beispiel 3, jedoch bei 200°C umgesetzt und aufgearbeitet. Die Ausbeute betrug 42%.

### Beispiel 5

Ethylencarbonat und Methylamin wurden wie in Beispiel 3, jedoch bei 300°C, umgesetzt und aufgearbeitet. Die Ausbeute betrug 45%.

## Patentansprüche

1. Verfahren zur Herstellung von Imidazolidin-2-onen der allgemeinen Formel (I) 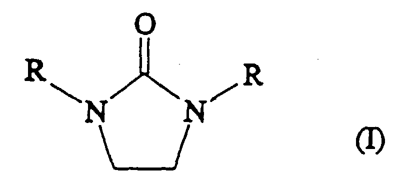 in der die Reste R, die gleich oder verschieden sein können,
a) geradkettige, verzweigte oder cyclische Alkylreste mit 1 bis 12 C-Atomen; die ihrerseits mit C₆₋₁₀-Aryl, F, Cl, Br substituiert sein können;
b) Arylreste mit 6 bis 10 C-Atomen, die ihrerseits mit C₁₋₁₂-Alkyl substituiert sein können;
c) Heteroalkylreste, in denen die wie oben definierten Alkylreste durch ein oder mehrere Heteroatome, ausgewählt aus O, S und N, unterbrochen sind,
d) Heteroarylreste mit 5 bis 10 Ringatomen, die 1 bis 3 Heteroatome, ausgewählt aus O, S, N, enthalten,
darstellen,
dadurch gekennzeichnet, daß man 1,3-Dioxalan-2-on (Ethylencarbonat) mit mindestens einer Verbindung der allgemeinen Formel (II)
RNH₂ (II)
umsetzt, in der R die vorstehende Bedeutung aufweist.

2. Verfahren nach Anspruch 1, dadurch gekennzeichnet, daß man die Umsetzung mit einer stöchiometrischen oder überstöchiometrischen Menge Amin, bezogen auf 1,3-Dioxalan-2-on, bevorzugt mit einem 1,5-bis 3fachen stöchiometrischen Überschuß, durchführt.

3. Verfahren nach Anspruch 1 oder 2, dadurch gekennzeichnet, daß man die Umsetzung bei einer Temperatur von 150 bis 300°C, bevorzugt 200 bis 250°C, durchführt.

4. Verfahren nach einem der Ansprüche 1 bis 3, dadurch gekennzeichnet, daß man die Umsetzung in Gegenwart von Wasser in einer Menge bis zu 50 Gew.-%, bevorzugt in einer Menge von 30 bis 45 Gew.-%, bezogen auf 1,3-Dioxalan-2-on, durchführt.

5. Verfahren nach einem der Ansprüche 1 bis 4, dadurch gekennzeichnet, daß man ein 1,3-disubstituiertes Imidazolidin-2-on unter Einsatz eines primären Amins der Formel (II), bei dem R bevorzugt jeweils Methyl ist, herstellt.

6. Verfahren nach einem der Ansprüche 1 bis 5, dadurch gekennzeichnet, daß man das Imidazolidin-2-on mit Hilfe einer Destillation gewinnt.

## Claims

1. A process for preparing 2-imidazolidinones of the general formula (I) 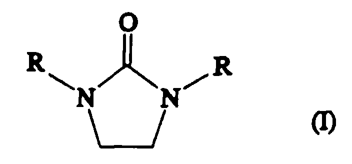 in which the radicals R, which can be identical or different, are
a) straight-chain, branched or cyclic alkyl radicals with 1 to 12 carbon atoms, which can in turn be substituted by C₆₋₁₀-aryl, F, Cl, Br;
b) aryl radicals with 6 to 10 carbon atoms, which can in turn be substituted by C₁₋₁₂-alkyl;
c) heteroalkyl radicals in which the alkyl radicals as defined above are interrupted by one or more heteroatoms selected from 0, S and N,
d) heteroaryl radicals with 5 to 10 ring atoms which contain 1 to 3 heteroatoms selected from 0, S, N,
which comprises reacting 1,3-dioxalan-2-one (ethylene carbonate) with at least one compound of the general formula (II)
RNH₂ (II)
where R has the above meaning.

2. A process as claimed in claim 1, wherein the reaction is carried out with a stoichiometric or more than stoichiometric amount of amine relative to 1,3-dioxalan-2-one, preferably with a 1.5 to 3 × stoichiometric excess.

3. A process as claimed in claim 1 or 2, wherein the reaction is carried out at from 150 to 300°C, preferably 200 to 250°C.

4. A process as claimed in any of claims 1 to 3, wherein the reaction is carried out in the presence of water in an amount of up to 50% by weight, preferably in an amount of from 30 to 45% by weight, based on 1,3-dioxalan-2-one.

5. A process as claimed in any of claims 1 to 4, wherein a 1,3-disubstituted 2-imidazolidinone is prepared using a primary amine of the formula (II) where R is preferably methyl in each case.

6. A process as claimed in any of claims 1 to 5, wherein the 2-imidazolidinone is isolated by distillation.

## Revendications

1. Procédé de préparation d'imidazolidine-2-ones de formule générale (I) 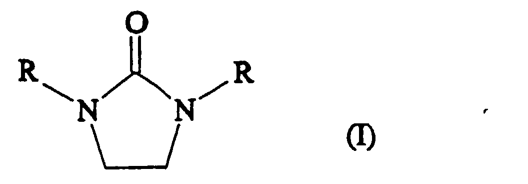 dans laquelle les radicaux R, qui peuvent être identiques ou différents, représentent :
a) des groupes alkyle cycliques ou à chaîne droite ou ramifiée, ayant de 1 à 12 atomes de carbone, et qui pour leur part peuvent être substitués par des groupes aryle en C₆₋₁₀, Cl, Br ;
b) des groupes aryle ayant de 6 à 10 atomes de carbone, qui pour leur part peuvent être substitués par des groupes alkyle en C₁₋₁₂ ;
c) des groupes hétéroalkyle, dans lesquels les groupes alkyle définis comme ci-dessus sont interrompus par un ou plusieurs hétéroatomes choisis parmi O, S et N ;
d) des groupes hétéroaryle ayant de 5 à 10 atomes nucléaires, qui contiennent de 1 à 3 hétéroatomes choisis parmi O, S, N,
caractérisé en ce qu'on fait réagir de la 1,3-dioxolanne-2-one (carbonate d'éthylène) avec au moins un composé de formule générale (II)
RNH₂ (II)
dans laquelle R a les significations données ci-dessus.

2. Procédé selon la revendication 1, caractérisé en ce qu'on met en oeuvre la réaction avec une quantité d'amine stoechiométrique ou supérieure à la quantité stoechiométrique, rapportée au 1,3-dioxolanne-2-one, et de préférence selon un excès de 1,5 à 3 fois la quantité stoechiométrique.

3. Procédé selon la revendication 1 ou 2, caractérisé en ce qu'on met en oeuvre la réaction à une température de 150 à 300°C, de préférence de 200 à 250°C.

4. Procédé selon l'une des revendications 1 à 3, caractérisé en ce qu'on met en oeuvre la réaction en présence d'eau en une quantité allant jusqu'à 50 % en poids, de préférence en une'quantité de 30 à 45 % en poids, par rapport à la 1,3-dioxolanne-2-one.

5. Procédé selon l'une des revendications 1 à 4, caractérisé en ce qu'on prépare une imidazolidine-2-one 1,3-disubstituée, par utilisation d'une amine primaire de formule (II) dans laquelle R est dans chaque cas de préférence le groupe méthyle.

6. Procédé selon l'une des revendications 1 à 5, caractérisé en ce qu'on obtient l'imidazolidine-2-one à l'aide d'une distillation.
